# EUROPÄISCHE PATENTSCHRIFT

(11) **EP 1 378 232 B1**
(45) Veröffentlichungstag und Bekanntmachung des Hinweises auf die Patenterteilung: **27.09.2006**
(21) Anmeldenummer: 03012747.6
(22) Anmeldetag: 04.06.2003
(51) Int. Cl.: A61K 8/92, A61Q 19/00

(54) **Mittel zur äusserlichen Anwendung beim Menschen**
Composition for external use
Composition pour application externe

(30) Priorität: 05.06.2002 DE 10225093; 28.05.2003 DE 10325159
(43) Veröffentlichungstag der Anmeldung: 07.01.2004
(73) Patentinhaber: Ziegler-Janoschka, Grazyna, 75177 Pforzheim (DE)
(72) Erfinder: Ziegler-Janoschka, Grazyna, 75177 Pforzheim (DE)
(74) Vertreter: Twelmeier, Ulrich

(56) Entgegenhaltungen:
- WO-A-98/40086
- CH-A- 688 787
- DD-A- 297 060
- FR-A- 2 262 012
- FR-A- 2 792 831
- US-A- 5 997 889
- US-B1- 6 368 639
- DATABASE WPI Section Ch, Week 198936 Derwent Publications Ltd., London, GB; Class B04, AN 1989-258971 XP002260599 & JP 01 186824 A (HORIUCHI ITARO SHOTEN KK) 26. Juli 1989 (1989-07-26)
- DATABASE WPI Section Ch, Week 199528 Derwent Publications Ltd., London, GB; Class B04, AN 1995-209370 XP002260600 & HU 67 367 A (MOLNAR G) 28. März 1995 (1995-03-28)

## Beschreibung

Die Erfindung geht von einem Mittel mit den im Oberbegriff des Anspruchs 1 angegebenen Merkmalen aus. Ein solches Mittelist aus DATABASE WPI Section Ch, Week 199528 Derwent Publications Ltd., London, GB; Class B04, AN 1995-209370 XP002260600 & HU 67 367 A (MOLNAR G) 28. März 1995 (1995-03-28) zur Vergrößerung der weiblichen Brust bekannt und enthält in 7 - 95 % eines Trägeröls Geraniumöl, Jasminöl, Ylang-Ylang-01, Carrotöl, Wachs, Vitamine A und E und Liposome.

Aus der US 6,368,639 B1 ist ein Hautpflegemittel bekannt, welches in Kombination mit weiteren Inhaltsstoffen auch Vetiveröl enthält.

Die Erfindung bezweckt die Bereitstellung eines Mittels zur Behandlung und Pflege der menschlichen Haut, welches durch eine neue Zusammensetzung eine verbesserte Wirkung hinsichtlich einer Straffung und Glättung der Haut und allgemein zur Verschönerung der Haut haben soll.

Diese Aufgabe wird gelöst durch ein Mittel, mit den im Anspruch 1 angegebenen Merkmalen. Überraschenderweise hat es sich gezeigt, dass ein solches Mittel ein unerwartet breites Wirkungsspektrum hat:
◆ Es eignet sich zur täglichen Pflege der gesamten Haut, mit Ausnahme der Schleimhäute.
◆ Es eignet sich zur Straffung der Haut, insbesondere der Haut im Gesicht und am Hals. Schon nach einer kurzen Anwendungsdauer von zum Beispiel zwei Wochen kann eine spürbare Straffung und Glättung der Haut beobachtet werden.
◆ Es eignet sich zur Verminderung von Zellulitis.
◆ Es eignet sich zur Verminderung von Schwangerschaftsstreifen.
◆ Es kann Hautunreinheiten verringern oder beseitigen.
◆ Es kann Linderung bringen bei Ekzemen und Neurodermitis.
◆ Es verbessert ganz allgemein den Zustand der Haut und hilft insbesondere gegen trockene und schuppige Haut.
◆ Bei regelmäßiger Anwendung auf der weiblichen Brust kann eine Vergrößerung der Brust beobachtet werden.
◆ Bei regelmäßiger Anwendung des Mittel kann auf andere Hautpflegemittel, wie zum Beispiel Cremes und Lotionen verzichtet werden.

Als Grundlage hat das Mittel ein Mandelöl, insbesondere ein kaltgepresstes süßes Mandelöl, welches sich durch eine besondere Hautverträglichkeit und einen angenehmen Geruch auszeichnet und von der Haut gut aufgenommen wird. Andere Pflanzenöle, zum Beispiel Olivenöl, kommen in der Summe ihrer Eigenschaften nicht an das süße Mandelöl heran, zum Beispiel weil sie stärker fetten oder nicht so bereitwillig in die Haut einziehen oder nicht so gut riechen usw.

Geraniumöl, Vetiveröl und Ylang-Ylang-ÖI sind ätherische Öle und entfalten ihre günstige Wirkung in geringen Konzentrationen. Vorzugsweise sind sie in dem Mittel in annähernd gleichen Volumenanteilen enthalten. Das ist insbesondere günstig für das breite Wirkungsspektrum, welches die Kombination dieser Inhaltsstoffe in dem erfindungsgemäßen Mittel aufweist. Außerdem hat der Einsatz des Geraniumöls, des Vetiveröls und des Ylang-Ylang Öls in annähernd gleichen Volumenanteilen den Vorteil, dass diese sich in ihren Wirkungen ausgewogen ergänzen und verstärken und dass keines von ihnen den Geruch des Mittels stark dominiert, sondern der Geruch aus den Bestandteilen besonders angenehm komponiert ist. Auf 1000 ml der Grundlage des Mittels werden lediglich bis zu 10 ml eines jeden der Inhaltsstoffe Geraniumöl, Vetiveröl und Ylang-Ylang-Öl eingesetzt. Größere Mengen der Inhaltsstoffe verstärken die günstigen Wirkungen des Mittels nicht, wohl aber seinen Geruch, der bei größeren Mengen als zu intensiv empfunden werden kann. Vorzugsweise werden auf 1000 ml des Mandelöls, welches vorzugsweise ein kaltgepresstes süßes Mandelöl ist, sogar nur bis zu 7 ml eines jeden der Inhaltsstoffe Geraniumöl, Vetiveröl und Ylang-Ylang-Öl eingesetzt. Bei zu geringen Mengen der Inhaltsstoffe verringert sich die Wirkung des Mittels. Deshalb soll das Mittel auf 1000 ml des Mandelöls wenigstens 4 ml eines jeden der Inhaltsstoffe Geraniumöl, Vetiveröl und Ylang-Ylang-Öl aufweisen.

Besonders bewährt hat sich ein Mittel, bei welchem auf
1000 ml süßes, kaltgepresstes Mandelöl
5,4 ml Geraniumöl,
5,4 ml Vetiveröl und
5 ml Ylang-Ylang-Öl
eingesetzt werden. Die ätherischen Öle werden nach bekannten Extraktionsverfahren aus den entsprechenden Pflanzen oder Pflanzenteilen hergestellt und sind als solche im Handel erhältlich.

Für den Handel eignet sich besonders die Zubereitung in Mengen von 250 ml wie folgt:
250 ml kaltgepresstes süßes Mandelöl
25 Tropfen Ylang-Ylang-ÖI (ungefähr 1,25 ml)
27 Tropfen Vetiveröl (ungefähr 1,35 ml)
27 Tropfen Geraniumöl (ungefähr 1,35 ml).

Für die Anwendung empfiehlt sich folgende Vorgehensweise:

Vor Gebrauch gut schütteln!
Die Anwendung ist einfach und kann für den ganzen Körper erfolgen. Man gibt etwas von dem zusammengesetzten Öl auf die Hand und massiert es in die gewünschten Körperteile ein, vorzugsweise einmal am Tag.

Zwecks Straffung des Busens massiert man diesen vorzugsweise zweimal täglich (am besten morgens und abends) 10 bis 15 Minuten lang mit dem Öl kräftig ein.

Ölreste kann man dann auf das Gesicht auftragen und einmassieren.
Bei regelmäßiger Anwendung des Mittels mit den ätherischen Ölen, die aus Blüten, Blättern und Wurzeln gewonnen werden, kann auf zusätzliche Cremes oder Lotionen verzichtet werden.

Ein Kontakt mit Augen und Schleimhäuten soll unbedingt vermieden werden.

Das Mittel und seine Bestandteile sind nicht zu inneren Anwendung bestimmt. Ein erfindungsgemäßes Mittel auf der Basis von Mandelöl mit den ätherischen Geranium-, Vetiver und Ylang-Ylang-Ölen ist das beste aus der Natur für das Wohlbefinden und für eine schöne Haut.

Ein erfindungsgemäßes Mittel kann Hilfsmittel zum Haltbarmachen und zum Dispergieren enthalten. Doch ist das nicht bevorzugt.

## Patentansprüche

1. Mittel zur äußerlichen Anwendung beim Menschen, welches in einem Öl als Grundlage als weitere Inhaltsstoffe Geraniumöl und Ylang-Ylang-Öl enthält, **dadurch gekennzeichnet, dass** die Grundlage ein Mandelöl ist, dass als weiterer Inhaltsstoff nur noch Vetiveröl enthalten ist und dass das Geraniumöl, das Vetiveröl und das Ylang-Ylang-Öl in Volumenanteilen von 4 ml bis zu 10 ml eines jeden der Inhaltsstoffe Geraniumöl, Vetiveröl und Ylang-Ylang-Öl in 1000 ml Mandelöl enthalten sind.

2. Mittel nach Anspruch 1, **dadurch gekennzeichnet, dass** die Grundlage ein süßes Mandelöl ist.

3. Mittel nach Anspruch 1 oder 2, **dadurch gekennzeichnet, dass** die Grundlage ein kaltgepresstes Mandelöl ist.

4. Mittel nach einem der Ansprüche 1 bis 3, **dadurch gekennzeichnet, dass** auf 1000 ml des Mandelöls bis zu 7 ml eines jeden der Inhaltsstoffe Geraniumöl, Vetiveröl und Ylang-Ylang-Öl eingesetzt werden.

5. Mittel nach einem der vorstehenden Ansprüche, **dadurch gekennzeichnet, dass** auf 1000 ml des Mandelöls 5,4 ml Geraniumöl, 5,4 ml Vetiveröl und 5 ml Ylang-Ylang-ÖI eingesetzt werden.

6. Verwendung eines Mittels nach einem der vorstehenden Ansprüche zur Hautpflege.

7. Verwendung eines Mittels nach einem der Ansprüche 1 bis 5 zur Straffung der Haut und zum Vermindern von Hautfalten.

8. Verwendung eines Mittels nach einem der Ansprüche 1 bis 5 zur Verminderung von Schwangerschaftsstreifen.

9. Verwendung eines Mittels nach einem der Ansprüche 1 bis 5 zur Verminderung von Zellulitis.

## Claims

1. Preparation for the external application on human beings which comprises an oil as a basis and comprises geranium oil and Ylang-Ylang oil as further ingredients in the oil basis, **characterized in that** the basis is an almond oil, that as a further ingredient there is provided vetiver oil, only, and that the preparation comprises in 1000 ml of the almond oil 4 ml to 10 ml of each of the ingredients geranium oil, vetiver oil and Ylang-Ylang oil.

2. Preparation according to claim 1, **characterized in that** the basis is a sweet almond oil.

3. Preparation according to claim 1 or 2, **characterized in that** the basis is a cold-pressed almond oil.

4. Preparation according to any of claims 1 to 3, **characterized in that** to 1000 ml of the almond oil there are added up to 7 ml each of the ingredients geranium oil, vetiver oil and Ylang-Ylang oil.

5. Preparation according to any of the preceding claims, **characterized in that** to 1000 ml of the almond oil there are added 5.4 ml of geranium oil, 5.4 ml of vetiver oil and 5 ml of Ylang-Ylang oil.

6. Using a preparation according to any of the preceding claims for skin care.

7. Using a preparation according to any of claims 1 to 5 for obtaining a smooth skin and to reduce skin folds.

8. Using a preparation according to any of claims 1 to 5 to reduce pregnancy stretch marks.

9. Using a preparation according to any of claims 1 to 5 to reduce cellulite.

## Revendications

1. Agent pour l'application externe chez l'être humain qui contient, dans une huile faisant office de substrat, à titre de constituants supplémentaires, de l'huile essentielle de géranium et de l'huile essentielle de Ylang-ylang, **caractérisé en ce que** le substrat est constitué d'une huile d'amande, **en ce que** l'agent contient, à titre de constituant supplémentaire, uniquement de l'huile essentielle de vétiver, et **en ce que** l'huile essentielle de géranium, l'huile essentielle de vétiver et l'huile essentielle de Ylang-ylang sont présentes dans des fractions volumiques de 4 ml à 10 ml pour chacun des constituants d'huile essentielle de géranium, d'huile essentielle de vétiver et d'huile essentielle de Ylang-ylang dans 1000 ml d'huile d'amande.

2. Agent selon la revendication 1, **caractérisé en ce que** le substrat est une huile d'amande douce.

3. Agent selon la revendication 1 ou 2, **caractérisé en ce que** le substrat est une huile d'amande pressée à froid.

4. Agent selon l'une quelconque des revendications 1 à 3, **caractérisé en ce que**, pour 1000 ml de l'huile d'amande, on met en oeuvre jusqu'à 7 ml de chacun des constituants d'huile essentielle de géranium, d'huile essentielle de vétiver et d'huile essentielle de Ylang-Ylang.

5. Agent selon l'une quelconque des revendications précédentes, **caractérisé en ce que**, pour 1000 ml de l'huile d'amande, on met en oeuvre 5,4 ml d'huile essentielle de géranium, 5,4 ml d'huile essentielle de vétiver et 5 ml d'huile essentielle de Ylang-Ylang.

6. Utilisation d'un agent selon l'une quelconque des revendications précédentes, pour l'entretien de la peau.

7. Utilisation d'un agent selon l'une quelconque des revendications 1 à 5, pour le raffermissement de la peau et pour la réduction des plis cutanés.

8. Utilisation d'un agent selon l'une quelconque des revendications 1 à 5, pour la réduction du masque de grossesse.

9. Utilisation d'un agent selon l'une quelconque des revendications 1 à 5, pour la réduction de la cellulite.
